# EUROPEAN PATENT SPECIFICATION

(11) **EP 2 872 613 B1**
(45) Date of publication and mention of the grant of the patent: **02.12.2020**
(21) Application number: 14703859.0
(22) Date of filing: 11.02.2014
(51) Int. Cl.: C11D 17/00, C11D 17/04, C11D 3/22

(54) **NEW SANITARY COMPOSITION**
NEUE HYGIENEZUSAMMENSETZUNG
NOUVELLE COMPOSITION HYGIÉNIQUE

(30) Priority: 10.05.2013 EP 13167356
(43) Date of publication of application: 20.05.2015
(73) Proprietor: Eurvest S.A., 1400 Nivelles (BE)
(72) Inventor: SIKORSKA, Agnieszka, 21-044 Trawniki (PL); LUCIANI, Alain, 13390 Auriol (FR); SAJ, Gabriela, 22-300 Krasnystaw (PL)
(74) Representative: August Debouzy
(86) International application number: PCT/EP2014/052673
(87) International publication number: WO 2014/180579

(56) References cited:
- EP-A1- 1 978 080
- EP-A1- 2 141 221
- WO-A1-98/46712
- WO-A1-2009/105233

## Description

### FIELD OF THE INVENTION

The present invention relates to sanitary compositions for cleaning and/or disinfecting and/or deodorizing sanitary elements, especially toilets.

### TECHNICAL BACKGROUND

Several solutions exist for cleaning and/or disinfecting and/or deodorizing sanitary elements, especially toilets.

An existing solution involves a block composition or a liquid composition which is placed in cage or either a specific delivery system and attached to the wall of the basin. Another solution involves a gelified liquid which is directly applied over the faience of the toilet, where the liquid is substantially viscous. Yet another solution is an adhesive gel that is applied locally to the toilet and is successively spread into the water of the basin with the flushes. The present invention deals with this last technical solution.

Different publications deal with such adhesive compositions.

For example, EP-A-1086199 discloses a sanitary product for cleaning and/or disinfecting and/or deodorizing, which product is said to be applicable directly to the sanitary ware, to be adhering there and only washable off after a large number of flushes, said product comprising water, anionic and/or nonionic and/or amphoteric surfactant, fragrances, an adhesion promoter selected from a wide list (comprising *inter alia* polyalkoxy alkanes, cellulose, polysaccharides, starch, alginates, diurethanes, gelatines, pectines, oleyl amines, alkyl dimethyl amine oxides, stearates, sodium dodecylbenzene sulfonates, agar agar, acacia gum, carob bean flour, polyacrylates, polyvinyl alcohols and polyvinylpyrrolidones). The compositions disclosed therein exhibit a specific viscosity of at least 15,000 mPas.

EP-A-1325103 is an improvement of the afore-mentioned patent application to the same applicant. The improvement lies in the use of specific polyhydroxy compounds.

WO-A-98/46712 discloses a gel sanitary composition comprising a polysaccharide (1-5%), a surfactant (3-25%) which is a C₈-C₂₂ alkyl polyglycolside, perfume (2-15%) and further additives. Xanthan gum and guar gums are examples of polysaccharides used in this patent application.

EP-A-0864637 also discloses a gel lavatory cleansing composition which permanently refreshes the air, in block form comprising surfactants, flush regulators, gel formers, fragrance (especially 0.5 to 20% by weight) and solvents. It is indicated that a certain fraction of the composition goes into solution during each flushing operation, such that the cleaning components are released continuously in the required amount. There is no indication about the adhesion properties.

EP1978080 discloses an adhesive composition comprising (i) an adhesion promoter which can be a polysaccharide, (ii) at least one surfactant, (iii) disinfecting and/or perfuming agents and optional compounds.

WO2009/105233 discloses an adhesive composition comprising (a) an adhesion promoter which can be a polysaccharide in an amount of 18 to 80%, (b) at least one anionic surfactant, e.g. alkylethersulfate, (c) mineral oil, (d) water and (e) a solvent which can selected from a list of glycols.

US2008/0255017 (corresponding to WO2006/056301) discloses an adhesive composition comprising an adhesion promoter which can be a polysaccharide, water, solvent, emulsifier and a bleaching agent.

There is no example with a polysaccharide in these documents, let alone a definition of what is a polysaccharide.

Current self-adhesive gels applied on the toilet as disclosed above, especially as described in EP-A-1325103 and/or EP-A-1086199 are based on the mixing of anionic surfactant with nonionic surfactant which thanks to Van der Waals bonds makes hexagonal net. This kind of binding provides a gel structure. However, when the product is in use (during flushing), those bonds are broken. This causes dissolution at the surface of the gel and therefore a poor visual aspect when the product is positioned in the toilets. This is seen as a negative by the end consumer.

US6846786 discloses a bar composition comprising (1) less than about 25% by wt. of surfactant; (2) greater than about 40% by wt. of sugar or mixture of sugars; (3) about 5% to 25% by wt. glass transition modifier; and (4) 1% to 30% water. The composition is said to be a bar.

EP0350306 discloses bars comprising 25-34% soap, 5-15% alcohol, 15-30% sugar, 15-30% water.

US3630925 discloses bars comprising 60-75% sugar, 20-25% soap, 2-5% germicidal agent.

There is a requirement for the product to be self-adhesive, and yet be transparent or translucent. It is also required that the sanitary compositions dissolve and disappear without leaving any residue or leakage on the wall.

### SUMMARY OF THE INVENTION

The composition of the invention can be used with or without cages, and in the latter case is directly applied on the toilet ware, even on wet surfaces.

Further, the compositions according to the instant invention exhibit excellent mechanical properties, since they do not require the use of a complex applicator and thus minimize the manipulations of the user.

The product obtained in the invention provides a self-adhesive paste, notably due to the ratio residual water/sugar, the surfactants providing no adhesive properties. This self-adhesive composition can be directly applied to the surface of the toilets while remaining sufficiently malleable to be easily processed and manipulated but at the same time sufficiently hard to last into the basin for a significant number of flushes and foam to ensure an efficient spread of the product into the basin. The resulting paste also provides enhanced aesthetical properties to the final product, including glossiness which allows lowering the amount of additives that are usually required to ensure an appropriate visual aspect to the public. The whitening effect can also be prevented with the new compositions according to the invention.

Hence, the invention provides a self-adhesive sanitary composition for cleaning and/or disinfecting and/or deodorizing in accordance with claim 1.

The invention also provides a process for the manufacture of a self-adhesive sanitary composition for cleaning and/or disinfecting and/or deodorizing in accordance with claim 12, and a composition obtainable thereby.

The invention also provides the use of the inventive composition for cleaning and/or disinfecting and/or deodorizing sanitary elements, especially toilets.

### DESCRIPTION OF EMBODIMENTS OF THE INVENTION

The present invention is disclosed in more details into the following description.

The invention is depicted in relation to the generic composition that is provided below as an example of a possible, non-limiting, composition.

The compositions according to the invention comprise a saccharide component, which can be any of a monosaccharide, disaccharide, or derivative thereof. The term "saccharide component" also includes any mixture of one or several of the previously cited components.

Monosaccharides are known as "ose", and comprise triose, tetrose, pentose, hexose, heptose. Preferred oses are hexoses, such as glucose, fructose, mannose, especially glucose. The monosaccharides within the meaning of the present invention also include compounds which include a saccharide moiety into their chemical structure such as a glucose moiety.

Disaccharides are also known and are formed of two "ose" bound by an osidic bound. Examples are maltose, lactose, cellubiose (reducing diholoside), and saccharose (also called sugar) and trehalose (non-reducing diholoside). The disaccharides within the meaning of the present invention also include compounds which derive from disaccharides such as saccharose.

The present invention further encompasses derivatives within the meaning of saccharides. The invention thus further provides for (di)saccharide derivatives, i.e. sugar alcohols which structure result from the replacement of one carbonyl by a hydroxyl group. Typically, one appropriate and preferred disaccharide derivative is isomalt, a sugar alcohol which derives from saccharose and comprises a glucose moiety in its chemical structure.

An oligosaccharide comprises up to 10 monomers; it is usually soluble which is preferred. Is preferably used a monosaccharide or disaccharide or a mixture thereof. Notably it can be a sugar. A preferred sugar is a mixture of saccharose/glucose syrup. A glucose syrup with increased amount of dextrose provides more adhesiveness/stickiness. A preferred amount of dextrose is 14-20%. Decreasing the amount of syrup generally decreases the adhesiveness/stickiness

More generally the preferred mixture of sugar is of the following chemical composition: X% (CₙH₂ₙOₙ)+ Y% (CₚH₂ₘOₘ) with n=6, p=12 and m<p and X+Y=100.

The overall amount of saccharide component within the compositions according to the invention typically represents 25 to 75 wt% of the total composition, preferably 40 to 70 wt%, and more preferably 40 to 65 wt%. The applicant has unexpectedly discovered that higher amounts of saccharides than what is usually involved in the formulation of sanitary compositions, (e.g. about 5 to 15 wt%) provides advantageous adhesive and dissolution properties when associated with high amounts of surfactant.

Mixtures of saccharide components are also envisaged. When glucose syrup is included into the composition, it may represent 30 to 90 wt% of the overall mixture of saccharide components and preferably 50 to 80 wt%, and more preferably 60 to 75 wt%. Usually glucose syrup is formulated with either saccharose (a disaccharide) or isomalt (a disaccharide derivative). These compounds exhibit advantageous properties in terms of dissolution.

The detergent action of the composition according to the invention Is exerted by a surfactant which can be any of an anionic, cationic, nonionic or amphoteric surfactant. Preferred surfactants are anionic.

The amount of surfactant into the composition typically amounts to above 25 wt% of the total composition, preferably between 30 and 50 wt% and more preferably between 30 and 40 wt%. As mentioned previously, the applicant has unexpectedly discovered that high proportions of surfactant in combination with high amounts of saccharides components yields to advantageous properties.

The amount of surfactant vs the amount of saccharide component is such that the weight ratio of the saccharide to the surfactant is from 3:1 to 1:1, preferably of about 2:1.

Substantially any surfactant can be used, i.e. anionic, cationic, nonionic, amphoteric, and mixtures thereof. Anionic surfactants are preferred since they exhibit excellent foaming, wetting, detergency, solubilizing, stabilizing and emulsifying functions.

Anionic surfactants can be:
(i) carboxylates (other than the fatty acids soaps used in the invention) such as alkyl carboxylates, aryl carboxylates, esters of carboxylic acids, ethers of carboxylic acids.
(ii) sulfates such as alkyl sulfates, e.g. ammonium lauryl sulfate (ALS), sodium lauryl sulfate (SLS, sodium dodecyl sulfate); MEA-alkyl sulfate; alkyl ether sulfates, e.g. sodium laureth sulfate, (sodium lauryl ether sulfate, SLES), sodium myreth sulfate; alkyl aryl ether sulfates; alkyl polyether glycerol ether sulfates, sulfated alkanolamides; glyceride sulfates.
(iii) sulfonates such as alkyl aryl sulfonates, e.g. dodecyl benzene sulfonate and synthetic detergents; alkyl sulfonates, e.g. alpha olefin sulfonates (AOS), primary and secondary parafin sulfonates (PS, SAS); sulfo fatty acids esters, e.g. FES (fatty esters sulfonate), MES (methyl ester sulfonate), ASME (a-sulfo methyl ester); sulfosuccinates, e.g. mono-alkyl sulfosuccinates, di-alkyl sulfosuccinates; fatty acid isethionates and taurides; lignosulfonates.
(iv) phosphoric acid, esters and salts, such as alkyl aryl ether phosphate; alkyl phenol ether phosphates; alkyl ether phosphate; alkyl phosphates.
(v) acylamino acids and salts, such as acyl glutamates, acyl peptides, acyl sarcosides.

A further possible list of anionic surfactant can be found at paragraphs [0056] to [0062], inclusive, of US2008/0255017 which is incorporated herein by reference. Preferred anionic surfactants are of the sulfate and/or sulfonate type.

Nonionic surfactants can be:
(i) fatty alcohols (alkanediols); such as cetyl alcohol, stearyl alcohol, cetostearyl alcohol (consisting predominantly of cetyl and stearyl alcohols), oleyl alcohol.
(ii) ethers; such as alkoxylated alcohols, especially ethoxylated alcohols, e.g. polyethyleneglycol (PEG), octaethylene glycol monododecyl ether, pentaethylene glycol monododecyl ether; EP/PO block polymer, e.g. ethoxylated PPG ether, polyoxypropylene glycol alkyl ethers, poloxamers; alkylpolyglucosides; ethoxylated oils and fats.
(iii) alkanolamides, such as mono- or dialkanolamide, ester-amide; ethoxylated alkanolamides, polyethoxylated monoalkanolamide esters, especially cocamide MEA.
(iv) ethoxylated fatty acids such as PEG fatty acid ester and PEG fatty acid diester.
(v) glycol esters, glycerol esters, and ethoxylated derivatives, such as ethylene glycol ester, propylene glycol ester, monoglyceride, 1,3-diglyceride, polyethoxylated 1,3-diglyceride, polyglyceryl monoester, glyceryl laurate.
(vi) sorbitan and sorbitol esters and ethoxylated derivatives, such as 1, 4-sorbitan monoester, sorbitan triester, polyethoxxlated sorbitan monoester, spans.
(vii) alkyl carbohydrate esters such as saccharose fatty acid monoester.
(viii) amine oxides such as dodecyldimethylamine oxide or coconut fatty acid amidopropyldimethylamine oxide.

A further possible list of nonionic surfactant can be found at paragraphs [0039] to [0050], inclusive, of US2008/0255017 which is incorporated herein by reference.

Cationic surfactants can be:
(i) alkyl amines, such as dimethyl alkyl amine and alkylamido dimethyl propylamine.
(ii) alkylimidazolines and imidazolines.
(iii) quaternary ammonium compounds such as tetraalkyl(-aryl) ammonium salts, heterocyclic ammonium salts, e.g. n-alkyl imidazoline chlorides, alkyl betaines such as coconut fatty acid amidopropylbetaine, ethoxylated alkyl amines e.g. alkyl propanediamine ethoxylate, esterified quaternaries e.g. esterquat.

Amphoteric surfactants can be:
(i) carboxylates, such as acyl ethylenediamines and derivatives, e.g. acylamphoacetate, acylamphodiacetate, acylamphodipropionate.
(ii) n-alkyl amino acids or imino diacids such as alkyl aminopropionic acid, sodium coco glycinate, aminopropyl alkylglutamide, sodium alkyliminodipropionate.
(iii) phosphates, such as lecithin.
(iv) sulfonates such as CHAPS (3-[(3-cholamidopropyl) dimethylammonio]-1-propanesulfonate).
(v) sultaine such as cocamidopropyl hydroxysultaine..

A solvent is generally added for the manufacture of the composition. It is water, or a mixture of water with an alcohol such as ethanol. Water is added in an amount from 10 to 25% by weight, preferably from 10 to 20%, based on the total weight of the composition. This amount is achieved by taking into account the amount of water (or mixture thereof with an organic solvent) included into the surfactant composition involved in the making of the compositions of the invention, the sugar side and generally speaking any water counted in the added material as well as added water. The amount of solvent, taking into account the non-aqueous solvent brought by the perfume, the dye, etc., would be up to 30%, preferably up to 28%. This selected amount of solvent allows obtaining a self-adhesive composition while at the same time enhances the stability and self-life of the product as well as its hardness. The final amount of water/solvent is much lower given evaporation that takes place during the manufacturing process.

Further commonly used components can be used as additives into the composition, such as stabilizers, fragrances/perfumes, dies, viscosity regulators, thickeners, disinfectants, enzymes, biocides, limescale removing agent, chelating agent, preservatives etc.

A stabilizer is generally used in the composition. It is generally used in amounts from 0.5 to 5% by weight, based on the total weight of the composition, preferably 1 to 3% by weight. The stabilizer is generally selected from the group consisting of:
- hydrophobic compounds, such as:
   - oils or waxes (animal, vegetable, synthetic, petroleum) typically liquid oils such as mineral or vegetable oils, preferably liquid paraffin and castor oil,
   - tertiary alcohols, ketones, complex esters.
- complexing agents, such as:
   - phosphoric acids, sodium diphosphates, stannetes, EDTA, NTA, citrates.
- urea, barbituric acid.
- hydrophobic and/or hydrophilic silica
- metal salts of fatty acids, such as
   - stearates salt (sodium, potassium).
- high temperature stable anionic surfactants, such as
   - 2-(N-erucacyl-N-methyl amido) acetate (EMAA), diphenyl oxide disulfonate, alkoxy carboxylate.
- silicate such as:
   - alkali metal silicate
- and silicon rubber.

A perfume is generally present in the composition. The perfume can be as disclosed at paragraph [0064] of US2008/0255017 which is incorporated herein by reference. The fragrance can be based on flowers fragrance, fruits, spices, and the like. The perfume can be of natural origin or can be synthetized. It is present generally in an amount from 2% to 10%, preferably from 3% to 6%.

A viscosity regulator (also known as flush regulator) can be present in the composition. As viscosity regulator, it can be chosen from the group of the mono or di - alcohols and polyols, and/or alkyl ethers. It can notably be a glycol such as propylene glycol (propane-1,2-diol ; 1,2-dihydroxypropane ; methyl glycol ; trimethyl glycol). Or it can be more generally a diol, such as 1,3 dihydroxypropane, 1,3 or 1,4 dihydroxybutane, 1,3 dihydroxyisobutane, or a polyol such as sorbitol or pentaerythritol. Alkyl ethers are possible, such as methyl, ethyl and n-butyl ether; for example it can be dipropylene glycol methyl ether. It can also be an alkanolamine such as diethanolamine. Preferably the viscosity regulator is absent, especially the composition is preferably free of PEG.

Thickeners can be used, such as xanthan gum, acacia gum, guar gum or carrageenan gum, or starch and/or derivatives, proteins (collagen, gelatin), pectin, PVA, silica, talc, and the like.

Various additional components known in the art of sanitary compositions can be added. The sanitary agent can thus comprise further customary constituents, for example disinfectants, enzymes, particles such as colored PET or PP, preservatives, biocides, etc. such as, for example, isothiazolone derivative, EDTA, or foam stabilizers, but also dyes or colorant and/or substances which dissolve limescale or urine scale in particular acids and/or phosphonates. The amount of additives is generally from 0% to 5%, preferably up to 3%.

The composition is manufactured according to the following process. In the main vessel the saccharide component or mixture thereof is introduced and melted at a temperature comprised between 100 and 160°C, e.g. 120 and 140°C. The additives, surfactants and optionally the solvent are mixed together in an auxiliary vessel and introduced at room temperature or after a preliminary heating up to 40°C (or up to 60 to 70°C) into the main vessel with the melted saccharide component. The thus resulting mixture is mixed during less than 10 minutes (e.g. 2 minutes) without additional heating up to a soft, homogeneous, consistency. The mixture is then poured to silicon/gum moulds and cooled down at room temperature or pass through cooling tunnel in certain time. During the manufacturing process, water introduced evaporates and the reaches the final amount of residual water. The final amount of residual water is measured according to EN ISO 9001:2008. This amount can be varied by adjusting the temperature or the amount (if any) of stabilizer such as the oil (e.g. liquid paraffinum). In the absence of oil for example, the temperature of the process can be lowered in order to reach the correct hardness and stickiness.

When the saccharide is a mixture of two or more, they can be added at separate steps during the process. Also, it is possible to add the viscosity regulator at different stages of the process.

Advantageously, the compositions are moulded in hydrodynamic shapes, to reach an optimized adhesiveness and use life of the product. Typically, the sanitary compositions according to the invention are symmetric and exhibit a smooth shape.

The composition is applied onto the toilet ware either directly, or according to known processes using any type of applicator, such as depicted in the afore-mentioned EP-A-1325103 and EP-A-1086199 and/or using the tools disclosed in the following patent applications WO-A-2007/008532, EP-A-2141221, WO-A-2011/135330 and EP-A-2281756.

The amounts are expressed by weight, unless indicated to the contrary.

The following examples illustrate the invention without limiting it.

### EXAMPLE

The compositions are manufactured as follows.
1. Sugar phase cook in 126-135°C.
2. Surfactant phase heat in microwave to 40-60°C.
3. Blend and mix together both phases to a soft consistency for about 2 minutes.

The following properties are tested. The disc has a round shape, of approximate dimensions as follows: diameter: 22-23mm, thickness: 10-11mm, weight: 4.5-5g.

Stickiness. Assess the level of stickiness of a toilet disc.

The methodology of stickiness test is taken from the patent US 2008/0190457. Used equipment was: strips of PET film and waxed paper of dimensions: long-5cm, wide-4cm, balance that weighs out to two decimal places and square ceramic tile. Values are given for both strips (note: in the US2008/0190457 patent application, stickiness to wax paper should be greater than 40 grams, while stickiness to balance surface should be greater than 80 grams.

Hardness. Assess the level of hardness of a toilet discs.

The equipment used was a Shore digital hardness tester manufactured by AFFRI System (parameters: measuring head for soft materials: a sponge, silicone rubber, etc.-Shore 00 scale; a ball indenter of 2.4 mm diameter; Press force 1,11 N; Time measurement mode: single measurement time 3 seconds; Temperature of measurement 24°C). The hardness test was done by pressing a ball penetrator into samples under the predetermined force. The hardness measurement is in tenths of a millimeter penetration into surface of samples.

Uselife test. Measure the duration of the product under conditions simulating reality of product use.

An automated cycle of 12 flushes per day distributed at irregular intervals (representative of the activity of a family of 3 persons) is triggered. The test is continued until complete consumption of the product that determines the duration of use. Operating time is expressed in number of flushes, which divided by 12, led to a period expressed in days.

Stability. Measure the stability in a given period of time.

Perform an accelerated stability corresponding to the 6 month shelf life, by using the standard ASTM F 1980. Accelerated storage conditions at 40°C are 30 days according to ASTM F 1980 and Arrhenius formula. Samples of products were stored in glass jars. Stability of products is checked by assessing visual aspect and measuring the dimensions.

The following compositions are manufactured.

The compositions are rigid, while plastic enough to be easily applied on the toilet. They are easy to apply on toilet and have excellent adhesive properties. Before flushing, the compositions are glossy with a nice visual aspect. This nice visual aspect is retained through the flushing. The life time exceed 100 flushes.

| Formula number → | | **1.1** | **1.2** | **1.3** | **1.4** | **1.5** |
|---|---|---|---|---|---|---|
| Component [%w/w] | | | | | | |
| Glucose syrup (dry content 80% | | 46 | 45 | 45 | 46 | 44,5 |
| Sugar | | 15 | 15 | 15 | | 14,5 |
| Isomalt | | | | | 15 | |
| Water | | 2 | 3 | 5 | 2 | 2 |
| Anionic surfactants (dry content 26.7-28%) | Sec. alkane sulphonate sodium salt (93% | 26 | 25 | 25 | 26 | 25 |
| | Alkyl ether sulfate C12 C14, sodium salt (70%) | 5 | 5 | 5 | 5 | 5 |
| Nonionic surfactants Cocamide MEA | | | | | | 3 |
| Liquid paraffinum | | 2 | | | 2 | 1 |
| Castor oil | | | 2 | | | |
| Fragrance | | 4.0 | 4.0 | 5.0 | 4 | 3 |
| DPG | | | | | | 2 |
| Dye | | 0.016-0.020 | | | | |

The following compositions are also manufactured.

| Formula number → | | **2.1** | **2.2*** | **2.3** | **2.4** |
|---|---|---|---|---|---|
| **Component [%w/w]** | | | | | |
| Glucamyl S651 Glucose syrup | | 46 | 46 | 47,5 | 46 |
| Sugar | | 15 | 15 | 12,5 | 15 |
| Water | | 2 | 2 | 2 | 2 |
| Anionic surfactants | Hostapur SAS 93 G Sodium C14/17 Sec Alkyl Sulphonate | 26 | 26 | 26 | 26 |
| | Texapon 70 Alky Ether Sulfate C12-C14, Sodium Salt | 5 | 5 | 5 | 5 |
| Liquid paraffinum | | 2 | 2 | 2 | 1 |
| Fragrance Blue Ocean 2 | | 4 | 4 | 5 | 5 |
| Dye | | 0.02 | 0.02 | 0.02 | 0.02 |
| **Residual water** | | 2.30 | 2.80 | 2.84 | 2.35 |

| **Stickiness test** | | | | | |
|---|---|---|---|---|---|
| **PET film** | | | | | |
| removing the strip of paper from the product | | 18 | 30 | 6 | 2 |
| removing the disc of product from the tile | | 170 | 160 | 230 | 250 |

| **Waxed paper** | | | | | |
|---|---|---|---|---|---|
| removing the strip of paper from the product | | 8 | 20 | 5 | 5 |
| removing the disc of product from the tile | | 230 | 180 | 250 | 160 |

| **Hardness test** | | | | | |
|---|---|---|---|---|---|
| Avarage | | 74,98 | 70,7 | 78,92 | 52,84 |
| Standard deviation | | 2,71 | 1,96 | 0,91 | 6,47 |

| **Uselife test** | | | | | |
|---|---|---|---|---|---|
| Life in days 8g @ 12flushes per day flower shape | | 160 | 160 | 180 | 140 |

| **Stability test** | | | | | |
|---|---|---|---|---|---|
| Visual inspection | | + | + | + | + |

| | | | | | |
|---|---|---|---|---|---|
| * sugar phase cooked in 124°C | | | | | |

Mono- and di-saccharide and optionally soluble oligosaccharide provide to the end product:
- adhesive properties
- structural properties (solid like product) and therefore do not require a specific applicator for its application into the toilet
- very attractive structure (similar to a candy) which makes the product much more attractive on shelves and in use.

The use of mono- and di-saccharide and optionally soluble oligosaccharide allows also better controlling the product dissolution over time. It can be used at high concentration to obtain the desired product structure while keeping a very good solubility in cold water.

Another advantage of mono- and di-saccharide and optionally soluble oligosaccharide is to easily adjust the product stickiness and product hardness through the processing parameter (e.g. processing temperature).

Mono- and di-saccharide also avoid the drying effect in use which leads to a kind of whitening effect at the surface of the product; mono- and di-saccharide being hydrophilic they behave as natural humectant, avoiding adding further ingredient in the formula, especially avoiding the additional use of glycerin.

## Claims

1. Self-adhesive sanitary composition for cleaning and/or disinfecting and/or deodorizing comprising, based on the total weight of the composition:
- a saccharide component selected from mono- or disaccharide, sugar alcohols or mixtures thereof, as an adhesion promoter in an amount expressed in dry content of from 40 to 65% by weight and
- a surfactant in an amount expressed in dry content of from 25 to 50 % by weight; and
- residual water in an amount from 1 to 5% by weight, based on the total weight of the composition.

2. Composition according to claim 1, comprising, by weight based on the total weight of the composition:
- 45 to 60 parts of the saccharide component;
- 26 to 40 parts of the surfactant;
- 1.5 to 3 % of water.

3. Composition according to anyone of claims 1 to 2, wherein the weight ratio of the saccharide to the surfactant is from 3:1 to 1:1, preferably of about 2:1.

4. Composition according to anyone of claims 1 to 3, wherein the mono- or disaccharide is glucose, fructose, saccharose, glucose syrup, isomalt or mixtures thereof, more preferably a mixture of saccharose and glucose syrup or a mixture of isomalt and glucose syrup, these mixtures being advantageously in weight ratios of 20/80 to 40/60.

5. Composition according to claim 4, wherein the dextrose content of the glucose syrup is from 14 to 20%.

6. Composition according to anyone of claims 1 to 5, wherein the surfactant is an anionic surfactant, typically an alkane sulphonate salt or an alkyl ether sulfate salt or a mixture thereof, preferably a mixture thereof.

7. Composition according to anyone of claims 1 to 6, further comprising a stabilizer, typically wherein the stabilizer is in amount of from 0.5 to 5%, preferably 1 to 3 wt% of the final composition.

8. Composition according to claim 7, wherein the stabilizer is selected from the group consisting of :
- hydrophobic compounds, such as oils or waxes (animal, vegetable, synthetic, petroleum) typically liquid oils such as mineral or vegetable oils, preferably liquid paraffin and castor oil, and tertiary alcohols, ketones, complex esters.
- complexing agents, such as phosphoric acids, sodium diphosphates, stannetes, EDTA, NTA, citrates.
- urea, barbituric acid.
- hydrophobic and/or hydrophilic silica
- metal salts of fatty acids, such as stearates salt (sodium, potassium).
- high temperature stable anionic surfactants, such as 2-(N-erucacyl-N-methyl amido) acetate (EMAA), diphenyl oxide disulfonate, alkoxy carboxylate.
- silicate such as alkali metal silicate
- and silicon rubber.

9. Composition according to anyone of claims 7 to 8, wherein the stabilizer is an oily substance, preferably liquid paraffinum or castor oil.

10. Composition according to anyone of claims 1 to 9 which exhibits a hardness of 50 to 80 tenths of a millimeter, preferably 65-75, measured at 24°C with a ball indenter of 2.4 mm diameter, a press force of 1.11 N and a measurement time of 3 seconds using Shore 00 scale.

11. Composition according to anyone of claims 1 to 10, which is devoid of glycerin.

12. A process for the manufacture of a self-adhesive sanitary composition for cleaning and/or disinfecting and/or deodorizing according to anyone of claims 1 to 11, which comprises melting a saccharide component, mixing with the remainder of the composition, optionally previously heated, and the moulding of the mixture, where the starting composition comprises, based on the total weight of the composition:
- a saccharide component selected from mono- or disaccharide, sugar alcohols or mixtures thereof, in an amount expressed as dry weight from 40% to 65% by weight of the total composition and
- a surfactant expressed as dry weight in an amount from 25 to 50% by weight of the total composition; and
- water in an amount from 10 to 25% by weight, preferably 10 to 20%,
wherein water is evaporated until a residual water content of 1 to 5% by weight is reached.

13. Composition obtainable by the process of claim 12.

14. Composition according to anyone of claims 1 to 11 or 13, in combination with a solid applicator.

15. Use of the composition according to any one of claims 1 to 11 and 13 or 14 for cleaning and/or disinfecting and/or deodorizing sanitary elements, especially toilets.

## Patentansprüche

1. Selbsthaftende Sanitärzusammensetzung zum Reinigen und/oder Desinfizieren und/oder Desodorieren, die in Bezug auf das Gesamtgewicht der Zusammensetzung umfasst:
- eine Saccharidkomponente, die aus Mono- oder Disaccharid, Zuckeralkoholen oder Mischungen davon ausgewählt ist, als haftungsförderndes Mittel in einer Menge von 40 bis 65 Gew.-% nach Trockengehalt, und
- ein Tensid in einer Menge von 25 bis 50 Gew.-% nach Trockengehalt; und
- Restwasser in einer Menge von 1 bis 5 Gew.-% in Bezug auf das Gesamtgewicht der Zusammensetzung.

2. Zusammensetzung nach Anspruch 1, die in Bezug auf das Gesamtgewicht der Zusammensetzung umfasst:
- 45 bis 60 Gewichtsteile Saccharidkomponente;
- 26 bis 40 Gewichtsteile Tensid;
- 1,5 bis 3 Gew.-% Wasser.

3. Zusammensetzung nach einem der Ansprüche 1 bis 2, wobei das Gewichtsverhältnis des Saccharids zu dem Tensid 3:1 bis 1:1, vorzugsweise ungefähr 2:1, beträgt.

4. Zusammensetzung nach einem der Ansprüche 1 bis 3, wobei das Mono-oder Disaccharid Glukose, Fruktose, Saccharose, Glukosesirup, Isomalt oder Mischungen davon ist, mehr bevorzugt eine Mischung aus Saccharose und Glukosesirup oder eine Mischung aus Isomalt und Glukosesirup, wobei diese Mischungen vorteilhafterweise in Gewichtsverhältnissen von 20/80 bis 40/60 vorliegen.

5. Zusammensetzung nach Anspruch 4, wobei der Dextrosegehalt des Glukosesirups 14 bis 20 % beträgt.

6. Zusammensetzung nach einem der Ansprüche 1 bis 5, wobei das Tensid ein anionisches Tensid ist, typischerweise ein Alkansulfonatsalz oder ein Alkylethersulfatsalz oder eine Mischung davon, vorzugsweise eine Mischung davon.

7. Zusammensetzung nach einem der Ansprüche 1 bis 6, die ferner einen Stabilisator umfasst, wobei der Stabilisator typischerweise in einer Menge von 0,5 bis 5 %, vorzugsweise 1 bis 3 Gew.-%, der Endzusammensetzung vorhanden ist.

8. Zusammensetzung nach Anspruch 7, wobei der Stabilisator aus der Gruppe ausgewählt ist, bestehend aus:
- hydrophoben Verbindungen wie z. B. Ölen oder Wachsen (tierisch, pflanzlich, synthetisch, Rohbenzin), typischerweise flüssigen Ölen wie z. B. Mineral- oder pflanzlichen Ölen, vorzugsweise flüssigem Paraffin und Rizinusöl, und tertiären Alkoholen, Ketonen, komplexen Estern.
- Komplexbildnern wie Phosphorsäuren, Natriumdiphosphaten, Stanneten, EDTA, NTA, Citraten.
- Harnstoff, Barbitursäure.
- hydrophobem und/oder hydrophilem Siliciumdioxid
- Metallsalzen von Fettsäuren wie Stearatsalzen (Natrium, Kalium).
- anionischen Tensiden mit hoher Temperaturstabilität wie z. B. 2-(N-Erucacyl-N-methylamido)acetat (EMAA), Diphenyloxiddisulfonat, Alkoxycarboxylat.
- Silikat wie z. B. Alkalimetallsilikat
- und Siliciumkautschuk.

9. Zusammensetzung nach einem der Ansprüche 7 bis 8, wobei der Stabilisator eine ölige Substanz ist, vorzugsweise flüssiges Paraffin oder Rizinusöl.

10. Zusammensetzung nach einem der Ansprüche 1 bis 9, die eine Härte von 50 bis 80 Zehnteln eines Millimeters, vorzugsweise 65 bis 75, aufweist, die bei 24 °C mit einem kugelförmigen Eindringkörper mit einem Durchmesser von 2,4 mm, einer Druckkraft von 1,11 N und einer Messzeit von 3 Sekunden auf einer Shore-00-Skala gemessen wurde.

11. Zusammensetzung nach einem der Ansprüche 1 bis 10, die kein Glyzerin aufweist.

12. Prozess zum Herstellen einer selbsthaftenden Sanitärzusammensetzung zum Reinigen und/oder Desinfizieren und/oder Desodorieren nach einem der Ansprüche 1 bis 11, der das Schmelzen einer Saccharidkomponente, das Mischen mit dem Rest der Zusammensetzung, der optional zuvor erhitzt wurde, und das Formgeben der Mischung umfasst, wobei die Ausgangszusammensetzung in Bezug auf das Gesamtgewicht der Zusammensetzung umfasst:
- eine Saccharidkomponente, die aus Mono- oder Disaccharid, Zuckeralkoholen oder Mischungen davon ausgewählt ist, in einer Menge nach Trockengewicht von 40 Gew.-% bis 65 Gew.-% der gesamten Zusammensetzung, und
- ein Tensid in einer Menge nach Trockengewicht von 25 bis 50 Gew.- % der gesamten Zusammensetzung; und
- Wasser in einer Menge von 10 bis 25 Gew.-%, vorzugsweise 10 bis 20 Gew.-%,
wobei Wasser verdampft wird, bis ein Restwassergehalt von 1 bis 5 Gew.- % erreicht ist.

13. Zusammensetzung, die mit dem Prozess nach Anspruch 12 erhältlich ist.

14. Zusammensetzung nach einem der Ansprüche 1 bis 11 oder 13 in Kombination mit einem Feststoffapplikator.

15. Verwendung der Zusammensetzung nach einem der Ansprüche 1 bis 11 und 13 oder 14 zum Reinigen und/oder Desinfizieren und/oder Desodorieren von Sanitärelementen, insbesondere Toiletten.

## Revendications

1. Composition sanitaire auto-adhésive pour le nettoyage et/ou la désinfection et/ou la désodorisation comprenant, sur la base du poids total de la composition :
- un composant saccharide choisi parmi un mono- ou disaccharide, les sucre alcools ou des mélanges de ceux-ci, en tant que promoteur d'adhésion en une quantité exprimée en teneur de matières sèches de 40 à 65 % en poids et
- un tensioactif en une quantité exprimée en teneur de matières sèches de 25 à 50 % en poids ; et
- de l'eau résiduelle en une quantité de 1 à 5 % en poids, sur la base du poids total de la composition.

2. Composition selon la revendication 1, comprenant, en poids sur la base du poids total de la composition :
- de 45 à 60 parties du composant saccharide ;
- de 26 à 40 parties du tensioactif ;
- de 1,5 à 3 % d'eau.

3. Composition selon l'une quelconque des revendications 1 à 2, dans laquelle le rapport en poids du saccharide au tensioactif est de 3:1 à 1:1, de préférence d'environ 2:1.

4. Composition selon l'une quelconque des revendications 1 à 3, dans laquelle le mono- ou disaccharide est le glucose, le fructose, le saccharose, le sirop de glucose, l'isomalt ou des mélanges de ceux-ci, plus préférablement un mélange de saccharose et de sirop de glucose ou un mélange d'isomalt et de sirop de glucose, ces mélanges étant avantageusement dans des rapports en poids de 20/80 à 40/60.

5. Composition selon la revendication 4, dans laquelle la teneur en dextrose du sirop de glucose est de 14 à 20 %.

6. Composition selon l'une quelconque des revendications 1 à 5, dans laquelle le tensioactif est un tensioactif anionique, typiquement un sel d'alcanesulfonate ou un sel d'alkyléther sulphate ou un mélange de ceux-ci, de préférence un mélange de ceux-ci.

7. Composition selon l'une quelconque des revendications 1 à 6, comprenant en outre un agent stabilisant, typiquement dans laquelle l'agent stabilisant est présent en une quantité de 0,5 à 5 %, de préférence de 1 à 3 % en poids de la composition finale.

8. Composition selon la revendication 7, dans laquelle l'agent stabilisant est choisi dans le groupe constitué par :
- les composés hydrophobes tels que les huiles ou les cires (animales, végétales, synthétiques, de pétrole), typiquement les huiles liquides telles que les huiles minérales ou végétales, de préférence une paraffine liquide et une huile de ricin, et les alcools tertiaires, les cétones, les esters complexes,
- les agents complexant, tels que les acides phosphoriques, les diphosphates de sodium, les stannètes, l'EDTA, le NTA, les citrates,
- l'urée, l'acide barbiturique,
- la silice hydrophobe et/ou hydrophile,
- les sels métalliques d'acides gras, tels qu'un sel de stéarate (sodium, potassium),
- les tensioactifs anioniques stables à haute température, tels que le 2-(N-erucacyl-N-méthylamido)acétate (EMAA), le disulfonate d'oxyde de diphényle, un alcoxycarboxylate,
- un silicate tels qu'un silicate de métal alcalin,
- et un caoutchouc de silicone.

9. Composition selon l'une quelconque des revendications 7 à 8, dans laquelle l'agent stabilisant est une substance huileuse, de préférence une paraffine liquide ou une huile de ricin.

10. Composition selon l'une quelconque des revendications 1 à 9 qui présente une dureté de 50 à 80 dixièmes d'un millimètre, de préférence de 65 à 75, mesurée à 24 °C avec un pénétrateur à billes de 2,4 mm de diamètre, une force de presse de 1,11 N et un temps de mesure de 3 secondes en utilisant une échelle Shore 00.

11. Composition selon l'une quelconque des revendications 1 à 10, qui est dépourvue de glycérine.

12. Procédé pour la fabrication d'une composition sanitaire auto-adhésive pour le nettoyage et/ou la désinfection et/ou la désodorisation selon l'une quelconque des revendications 1 à 11, qui comprend la fusion d'un composant saccharide, le mélange avec le reste de la composition, éventuellement préalablement chauffée, et le moulage du mélange, où la composition de départ comprend, sur la base du poids total de la composition :
- un composant saccharide choisi parmi un mono- ou disaccharide, les sucres alcools ou des mélanges de ceux-ci, en une quantité exprimée en teneur de matières sèches de 40 à 65 % en poids de la composition totale et
- un tensioactif exprimé en poids de matières sèches en une quantité de 25 à 50 % en poids de la composition totale ; et
- de l'eau en une quantité de 10 à 25 % en poids, de préférence de 10 à 20 %,
dans lequel l'eau est évaporée jusqu'à ce qu'une teneur en eau résiduelle de 1 à 5 % en poids soit atteinte.

13. Composition pouvant être obtenue par le procédé selon la revendication 12.

14. Composition selon l'une quelconque des revendications 1 à 11 ou 13, en combinaison avec un applicateur solide.

15. Utilisation de la composition selon l'une quelconque des revendications 1 à 11 et 13 ou 14 pour le nettoyage et/ou la désinfection et/ou la désodorisation d'éléments sanitaires, en particulier des toilettes.
